# EUROPEAN PATENT APPLICATION

(11) **EP 4 410 973 A1**
(43) Date of publication of application: **07.08.2024**
(21) Application number: 23765778.8
(22) Date of filing: 21.02.2023
(51) Int. Cl.: C12N 9/10, C12N 15/54, C12N 1/19, C12N 9/38, C12N 15/56, C12P 19/26, C12P 19/18, C12P 7/06, C12R 1/645

(54) **RECOMBINANT YEAST AND APPLICATION THEREOF**

(30) Priority: 11.03.2022 CN 202210234497; 21.11.2022 CN 202211453172
(71) Applicant: Shandong Henglu Biotech. Co., Ltd., Jinan, Shandong 250004 (CN)
(72) Inventor: FANG, Xu, Jinan, Shandong 250000 (CN); LI, Fengyi, Jinan, Shandong 250000 (CN); HAN, Lijuan, Jinan, Shandong 250000 (CN); YIN, Wencheng, Jinan, Shandong 250000 (CN)
(74) Representative: Herrmann, Uwe
(86) International application number: PCT/CN2023/077280
(87) International publication number: WO 2023/169200

(57) **Abstract**

Provided are a recombinant yeast for producing lacto-N-triose, and an application thereof, belonging to the bioengineering field. The recombinant yeast comprises an amino acid sequence of a β-N-acetylglucosaminyl transferase and a coding nucleic acid sequence thereof, and/or a disruption of a marker gene. The technical solution of the present invention has the advantages of easy availability of raw materials, simple and feasible method, suitable for industrial production, and excellent application prospects.

## Description

The present invention claims priority to the Chinese patent applications submitted to the China National Intellectual Property Administration on March 11, 2022, with the application number 202210234497.8, titled "recombinant yeast and application thereof, and on November 21, 2022, with the application number 202211453172.5, also titled "recombinant yeast and application thereof. The entire contents of these applications are incorporated herein by reference.

### TECHNICAL FIELD

The present invention relates to recombinant yeast for the production of lacto-N-triose and applications thereof, which belongs to the field of bioengineering.

### BACKGROUND

Human Milk Oligosaccharides (HMOs) are a type of complex oligosaccharides composed of monosaccharides, derivatives, sialic acids, and other structural units linked by glycosidic bonds.

Some HMOs-added products, such as infant formula milk powder and dietary supplements, have already been sold in the United States, the European Union, Australia, New Zealand and other countries.

Both the European Food Safety Authority (EFSA) and the US Food and Drug Administration (FDA) have approved lacto-N-tetraose (LNT) and lacto-N-neotetraose (LNnT) for marketing. These are produced using microbial fermentation technology.

Since the 21st century, with the advancement of research methods, scientists have discovered that the total concentration of HMOs (both acidic and neutral) in breast milk decreases as the lactation period extends; therefore, the timely and adequate supplementation of HMOs would be beneficial for maintaining the healthy growth of infants and young children. However, the synthesis of HMOs has always been a significant challenge faced by synthetic biologists and chemical engineers. From the perspective of synthesizing glycans within organisms, the production of such molecules is not a mere replication from a single template, but requires the coordinated regulation of multiple glycosyltransferases and glycosidases, which determines the complexity, diversity, and micro-heterogeneity of glycan structures.

Lacto-N-triose II (LNTII, also known as GlcNAc-β1, 3-Gal-β1, 4-Glc, CAS: 75645-27-1, with chemical structure as Formula 1).

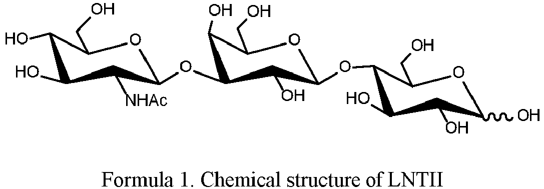

LNTII is not only an important oligosaccharide of HMOs, but also an essential precursor for the synthesis of Lacto-N-tetraose (LNT) and Lacto-N-neotetraose (LNnT). Even though some literatures have disclosed certain techniques or strategies for LNTII preparation, the complex structure of LNTII makes it challenging to control the yield, stereo-selectivity, and regio-selectivity during the synthesis. Therefore, it is crucial to have sustainable and safe production techniques for LNTII to be commercialized. Nyffenegger et al. reported that LNTII can be synthesized through enzymatic catalysis, forming a β-1,3-glycosidic bond between N-acetylglucosamine (GlcNAc) and β-lactose. (Backbone structures in human milk oligosaccharides: trans-glycosylation by metagenomic β-N-acetylhexosaminidases. Applied Microbiology and Biotechnology, 2015; 99: 7997-8009).

Biosynthesis technology has gained a lot of attention in recent years due to its advantages of high specificity and scalable mass production. Currently, there are two main methods for the synthesis of human milk oligosaccharides: cell culture and enzymatic catalysis. Although the enzymatic catalysis has advantages of strong controllability and less by-product reaction, the catalytic efficiency is low. For instance, patent CN202010386045.2 discloses the application of β-N-acetylhexosaminidase derived from *Haloferula sp.* in the synthesis of LNTII. It uses the substrate chitin as an amino sugar donor, but the conversion rate is less than 14%. Whole-cell catalysis directly utilizes high-energy activated substrates like UDP-GlcNAc, which is an easier method to achieve higher conversion rates. For example, Chinese patent CN201680052611.8 discloses a method for producing the desired oligosaccharides using genetically modified microbial host cells, while CN202010834657.3 reveals a genetically engineered bacterium and method for enhancing the production of LNTII. At present, *E*. *coli* is the most commonly used host organism for producing LNTII, but removing endotoxins from *E*. *coli* is a significant challenge for large-scale industrial production. Therefore, finding a host that can provide stable yields, high production efficiency, and good safety to synthesize LNTII is of great value for the industrial application and promotion of human milk oligosaccharides.

### SUMMARY

Objective of the present invention: in response to the existing technical challenges and problems, the present invention is to provide a safe, highly specific, and sustainable biosynthesis method.

Described herein is a microbial strain containing modifications related to a gene of β-N-acetylglucosaminyl transferase.

In one aspect, the present invention provides an enzyme for catalyzing the production of lacto-N-triose II (LNTII). According to the Carbohydrate-Active Enzymes (CAZy) database, the enzyme for catalyzing the production of lacto-N-triose II (LNTII) belongs to the glycoside hydrolase family 28. The enzyme of glycoside hydrolase family 28 is β-N-acetylglucosaminyl transferase.

The enzyme for catalyzing the production of LNTII is preferably derived from *Neisseria meningitidis,* under EC No: 2.4.1.56 and GenBank number AAC44084.1. An amino acid sequence of the enzyme is as set forth in SEQ ID NO:1, or comprises an amino acid sequence as set forth in SEQ ID NO:1, or has at least 70% amino acid sequence identity with SEQ ID NO:1, or comprises an amino acid sequence with at least 70% amino acid sequence identity with SEQ ID NO: 1.

The enzyme for catalyzing the production of LNTII is preferably derived from *Helicobacter pylori* under EC No: 2.4.1.129 and GenBank number WP000199766.1. An amino acid sequence of the enzyme is as set forth in SEQ ID NO:2, or comprises an amino acid sequence as set forth in SEQ ID NO:2, or has at least 70% amino acid sequence identity with SEQ ID NO:2, or comprises an amino acid sequence with at least 70% amino acid sequence identity with SEQ ID NO:2.

The enzyme for catalyzing the production of LNTII is preferably derived from *Salmonella enterica* under EC No: 2.4.1.155 and GenBank number WP_140239824.1. An amino acid sequence of the enzyme is as set forth in SEQ ID NO:3, or comprises an amino acid sequence as set forth in SEQ ID NO:3, or has at least 70% amino acid sequence identity with SEQ ID NO:3, or comprises an amino acid sequence with at least 70% amino acid sequence identity to SEQ ID NO: 3.

The enzyme for catalyzing the production of LNTII includes enzymes comprising an amino acid sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity to SEQ ID NO: 1, SEQ ID NO: 2 or SEQ ID NO: 3 and has the activity to produce LNTII.

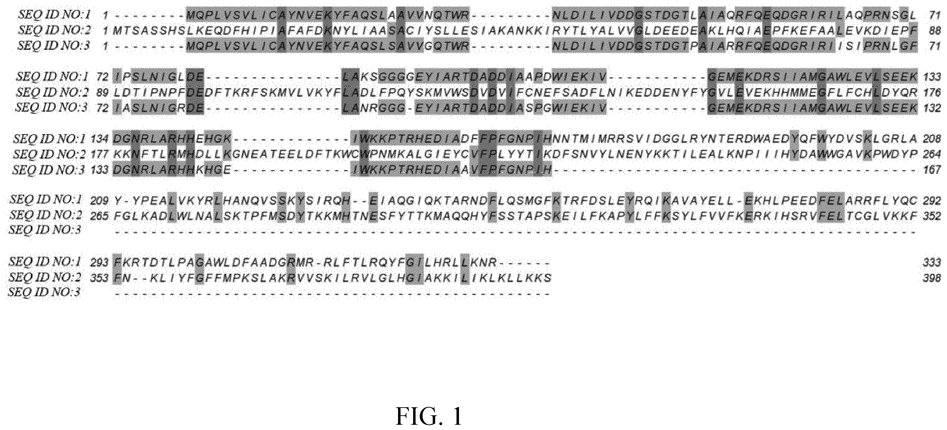

As shown in FIG. 1, there are significant differences among SEQ ID NO: 1, SEQ ID NO: 2, and SEQ ID NO: 3. The amino acid sequence identity between SEQ ID NO: 1 and SEQ ID NO: 2 is 12.5%, the amino acid sequence identity between SEQ ID NO: 1 and SEQ ID NO: 3 is 44.44%, and the amino acid sequence identity between SEQ ID NO: 2 and SEQ ID NO: 3 is 5.28%.

In some embodiments, the present invention describes a recombinant cell, comprising one or more (such as two or several) heterologous polynucleotides. Preferably, the heterologous polynucleotides are selected from those encoding β-N-acetylglucosaminyl transferase capable of producing LNTII.

The recombinant cell preferably comprises nucleic acid sequences that can encode enzymes for catalyzing the production of LNTII comprising amino acid sequences as shown in SEQ ID NO: 1 and/or SEQ ID NO: 2 and/or SEQ ID NO: 3.

In some embodiments, the present invention describes that a yeast cell is used as a host with the aid of its own metabolic pathway and the introduction of heterologous β-N-acetylglucosaminyl transferase of glycoside hydrolase family 28 to efficiently synthesize lactose-N-triose (Lac-N-Triose II, LNTII).

In some embodiments, the recombinant cell is a yeast cell, such as those from the genera *Candida, Hansenula, Kluyveromyces, Pichia, Saccharomyces, Schizosaccharomyces,* or *Yarrowia;* further preferably, cells from *Saccharomyces cerevisiae, Kluyveromyces lactis, Kluyveromyces marxianus, Yarrowia lipolytica, Rhodotorula graminis, Saccharomyces pastorianus,* etc. *Saccharomyces cerevisiae, Kluyveromyces lactis, Kluyveromyces marxianus,* and *Yarrowia lipolytica* are yeast cells that have been certified by the U.S. Food and Drug Administration GRAS, and are more easily accepted in terms of food or food additive safety management.

The present invention provides a novel production process for LNTII that can be synthesized easily through fermentation, which not only simplifies the synthesis steps but also enhances the safety of the target product.

In another aspect, the recombinant cell is designed to comprise (1) an β-N-acetylglucosaminyl transferase of LNTII, and (2) a disruption of a marker gene.

The term "disruption" means that the coding region and/or control sequence of a reference gene is partially or completely modified (e.g., by deletion, insertion, and/or substitution of one or more nucleotides) such that the expression of the encoded polypeptide is absent (inactivated) or reduced, and/or the enzymatic activity of the encoded polypeptide is absent or reduced. The effect of disruption can be measured using known techniques in the art, such as detecting the absence or reduction of lactose consumption using cell-free extract measurements referenced herein.

The marker gene includes a nucleotide sequence encoding all or part of the amino acid sequence of β-galactosidase. The amino acid sequence encoded by the marker gene has at least 50%, 51%, 52%, 53%, 54%, 55%, 60%, 61%, 62%, 63%, 64%, 65%, 66%, 67%, 68%, 69%, 70%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence identity to SEQ ID NO: 4 and/or SEQ ID NO: 5.

Preferably, the recombinant yeast is a recombinant *Kluyveromyces lactis* cell; further preferably, it includes at least one marker gene that is disrupted, and comprises an amino acid sequence of the marker, and a nucleic acid sequence encoding the marker. The nucleic acid sequence of the marker gene is registered under GenBank number M84410.1, and the amino acid sequence encoded thereby is registered under GenBank number AAA35265.1.

Preferably, the recombinant yeast is a recombinant *Kluyveromyces marxianus* cell; further preferably, it includes at least one marker gene that is disrupted, and comprises an amino acid sequence of the marker, and a nucleic acid sequence encoding the marker. The nucleic acid sequence of the marker gene is registered under GenBank number XM_022818497.1, and the amino acid sequence thereof is registered under GenBank number XP_022675157.1.

In one embodiment, the marker gene is β-galactosidase gene *lac4.* The disruption of the marker gene preferably refers to the disruption of the *lac4* gene. The disruption of the *lac4* gene preferably refers to the disruption of part of the marker gene.

The recombinant *Kluyveromyces lactis* strain is preferably the recombinant *Kluyveromyces lactis* strains KL-NMLgtA and/or KL-SELgtA and/or KL-HPLgtA.

The recombinant *Kluyveromyces marxianus* strain is preferably the recombinant *Kluyveromyces marxianus* strains KM-NMLgtA and/or KM-SELgtA and/or KM-HPLgtA.

Preferably, the recombinant *Kluyveromyces lactis* strain StrainKL-2-02 in the present invention is classified as *Kluyveromyces lactis,* deposited under accession number CCTCC NO: M 2022118.

Preferably, the recombinant yeast in the present invention can be genetically modified through ultraviolet mutagenesis to further increase the yield of LNTII.

The present invention also provides a method for constructing the recombinant yeast cell as described above, comprising: (a) culturing a parental strain; (b) disrupting a marker gene in the parent strain from (a); (c) introducing an enzyme capable of producing LNTII; preferably, further comprising (d) a step of isolating the recombinant yeast cell.

The term "parental" or "parental strain" refers to a strain that is subjected to disruption to produce the recombinant strain described herein. The parental can be a naturally occurring (wild-type) or a pre-modified strain.

Preferably, the yeast strain is a *Kluyveromyces sp.* strain; more preferably, the yeast strain is a *Kluyveromyces lactis* strain *K. lactic* and/or *Kluyveromyces marxianus* strain *K. marxianus;* further preferably, the yeast strain is the *Kluyveromyces lactis* strain *K. lactic* DSM70799 and/or *Kluyveromyces marxianus* strain *K. marxianus* DMKU3-1042.

An application of the recombinant *Kluyveromyces lactis* strain and the recombinant *Kluyveromyces marxianus* strain of the present invention for the production of LNTII is provided. The recombinant *Kluyveromyces lactis* strain and the recombinant *Kluyveromyces marxianus* strain utilize UDP-N-acetylglucosamine (UDP-GlcNAc) and lactose synthesized by their own metabolism as reaction donors, to produce LNTII under the catalysis of heterologously introduced glycoside hydrolases of glycoside hydrolase family 28.

The present invention also provides an application of the recombinant *Kluyveromyces lactis* strain and/or the recombinant *Kluyveromyces marxianus* strain for the production of LNTII.

Preferably, the medium used in the application is an aqueous solution; more preferably, the medium used in the application is an aqueous solution containing lactose and/or glucose and/or fructose and/or sucrose and/or galactose. Preferably, the aqueous solution requires the addition of the recombinant *Kluyveromyces lactis* strain and/or the recombinant *Kluyveromyces marxianus* strain.

Preferably, the culture temperature for the application is 15-60 °C; further preferably, the culture temperature for the application is 28-32 °C.

Preferably, in the application of the recombinant yeast for the production of LNTII, the medium also includes an inorganic salt(s) and/or a trace element(s). Preferably, the inorganic salt(s) and/or trace element(s) is/are selected from dipotassium hydrogen phosphate, magnesium sulfate, ammonium sulfate, manganese sulfate, potassium sulfate, or any combination thereof. Preferably, the final concentration of the inorganic salt(s) or trace element(s) is 3-7 mM.

The present invention also provides a method of producing LNTII, comprising (a) culturing the recombinant yeast cell in a fermentable medium under suitable conditions to produce LNTII; and (b) recovering the LNTII.

The method of producing LNTII preferably performs separation and purification to obtain LNTII by the addition of activated carbon, etc.

The method of producing LNTII preferably utilizes the recombinant yeast to perform mixed fermentation to obtain LNTII.

The explanation of terms: the term "sequence identity" refers to the correlation between two amino acid sequences or two nucleotide sequences, described by the parameter of "sequence identity", also known as "sequence homology".

The term "host cell" refers to any cell type that is susceptible to transformation, transfection, transduction, etc., with a nucleic acid construct or expression vector. The term "host cell" encompasses any progeny of a parental cell that is different from the parental cell due to mutations occurring during replication.

The term "recombinant cell" is defined as a non-naturally occurring host cell comprising one or more (e.g., two, or several) heterologous polynucleotides. The host cell can be a mutant strain as described herein or can be further disrupted to provide a mutant strain as described herein.

### Beneficial Technical Effects:

The present invention provides a novel method for the production of lacto-N-triose II (LNTII), utilizing recombinant *Kluyveromyces* yeasts (recombinant *Kluyveromyces lactis* and recombinant *Kluyveromyces marxianus*) as cell factories to synthesize LNTII in human milk oligosaccharides. Compared to previous methods, the entire production process is simple and controllable, and has high biosafety. It is easily expanded in scale, with a broad range of raw material sources, low product preparation costs, and high practical value. The conversion rate in the synthesis of lacto-N-triose II (LNTII) using lactose as a substrate is higher.

The present invention proves that recombinant yeast cells have tremendous potential for the efficient production of human milk oligosaccharides. It lays the foundation for the large-scale industrial production of lacto-N-triose II (LNTII) and analogs thereof, holding significant economic value and social benefits. In addition to safety, the raw materials used in the present invention are easily available, and the method is simple and feasible, is suitable for industrial production and has excellent application prospects.

### BRIEF DESCRIPTION OF THE DRAWINGS

FIG. 1. A comparison chart of the amino acid sequences shown in SEQ ID NO: 1, SEQ ID NO:2, and SEQ ID NO:3.
FIG. 2. High Performance Liquid Chromatography (HPLC) profiles of the synthetic products from the original strain of *Kluyveromyces lactis* and the control strain StrainKL-1 in Example 3.
FIG. 3. HPLC profiles of the synthetic products from the fermentation broth of *Kluyveromyces lactis* StrainKL-2-01 in Example 4, the fermentation broth of the recombinant strain StrainKL-2-02 in example 6 and the LNTII standard.
FIG. 4. HPLC profiles of the synthetic products from the purified fermentation broths of the recombinant *Kluyveromyces lactis* strain StrainKL-2-01 in Example 4 and the LNTII standard.
FIG. 5. HPLC profiles of the synthetic products from the purified fermentation broths of the recombinant *Kluyveromyces lactis* strain StrainKL-2-02 in Example 6 and the LNTII standard.
FIG. 6. Liquid Chromatography-Mass Spectrometry (LC-MS) profile of the LNTII standard.
FIG. 7. LC-MS profile of the product at a peak time of 12.10 min in the fermentation broth of the recombinant *Kluyveromyces lactis* strain StrainKL-2-02 in FIG. 5.
FIG. 8. Proton Nuclear Magnetic Resonance (¹H-NMR) spectrum of the product at a peak time of 12.10 min in the fermentation broth of the recombinant *Kluyveromyces lactis* strain StrainKL-2-02 in FIG. 5.

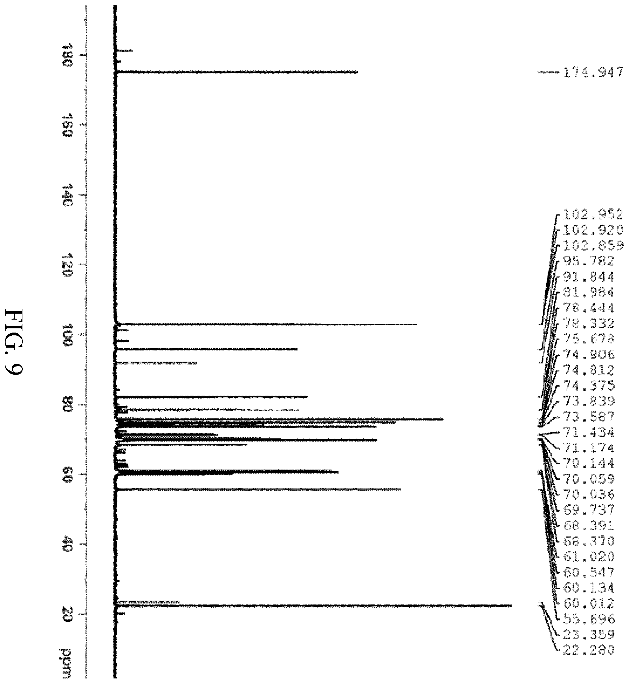

FIG. 9. Carbon-13 Nuclear Magnetic Resonance (¹³C-NMR) spectrum of the product at a peak time of 12.10 min in the fermentation broth of the recombinant *Kluyveromyces lactis* strain StrainKL-2-02 in FIG. 5.

### DETAILED DESCRIPTION

It should be noted that the following description and examples are illustrative and intended to provide further explanation of the present invention. Unless otherwise specified, all technical and scientific terms used herein have the same meaning as commonly understood by one of ordinary skill in the art to which the present invention belongs.

It is important to note that the terms used here are only for describing specific embodiments, and not intended to limit the exemplary embodiments according to the present invention. Furthermore, it should be understood that when the terms "comprises" and/or "comprising" are used in this specification, they specify the presence of stated features, steps, operations, devices, components, and/or combinations thereof. It should be understood that the scope of the present invention is not limited to the specific embodiments described below; it should also be understood that the terms used in the examples of the present invention are for describing specific embodiments, not for limiting the scope of the present invention. The experimental methods in the following specific embodiments, if not specified, are generally performed according to conventional methods and conditions in the field of molecular biology, as fully explained in the literature. For example, see the techniques and conditions described in Sambrook et al., Molecular Cloning: A Laboratory Manual*,* or as recommended by the manufacturer of the reagents.

The following examples further explain the present invention but do not constitute a limitation of the present invention. It should be understood that these examples are used to illustrate the present invention and not to limit the scope of the present invention. The experimental methods in the following examples, if not specified, are generally performed under conventional conditions.

In the specific embodiments of the present invention, *Kluyveromyces lactis* and/or *Kluyveromyces marxianus,* which have good biosafety and are widely used in industry, were used as hosts. To prevent lactose consumption within the yeast strain, the β-galactosidase gene *lac4* of *Kluyveromyces lactis* and/or *Kluyveromyces marxianus* was knocked out; genes encoding acetylglucosaminyltransferase from *Neisseria meningitidis* NMLgtA and/or from *Salmonella enterica* (SELgtA) and/or from *Helicobacter pylori* (HPLgtA) were respectively integrated into the genome of *Kluyveromyces lactis* or *Kluyveromyces marxianus* to obtain a strain that synthesizes lactose-N-triose (LNTII).

The yeast cells undergo a two-stage cultivation process. In the first stage, the cells are cultured using glucose as the carbon source. This helps in the accumulation of biomass and enzymes until the cell growth reaches the late logarithmic or stationary phase. In the second stage, the product synthesis phase begins. Initially, the culture is shaken at 30°C and 200 rpm for 40 hours. Then after, 2 g/L of lactose, 20 g/L of glucose, and a final concentration of 3-7 mM of metal ions are added to the medium. This ultimately leads to the production of LNTII.

The proteins produced in the host cells can be obtained by any known process for protein separation and purification.

These processes include, but are not limited to, salt precipitation and solvent precipitation, ultrafiltration, gel electrophoresis, ion exchange chromatography, affinity chromatography, reverse-phase high-performance liquid chromatography, hydrophobic interaction chromatography, mixed mode chromatography, hydroxyapatite chromatography, and isoelectric focusing.

The specific embodiments of the present invention also provide a method for synthesizing LNTII.

Specifically, the method includes: amplifying the genes encoding β-N-acetylglucosaminyl transferase from *Neisseria meningitidis, Salmonella enterica,* and *Helicobacter pylori* and constructing expression cassettes suitable for *Kluyveromyces lactis* and/or *Kluyveromyces marxianus;* introducing the expression cassettes into the genome of *Kluyveromyces lactis* DSM70799 and/or *Kluyveromyces marxianus* DMKU3-1042 strains; selecting positive yeast transformants; fermenting the genetically engineered strain to produce LNTII; identifying the fermentation broth and its purified products using high-performance liquid chromatography, mass spectrometry, and nuclear magnetic resonance spectroscopy.

In the following examples, *Escherichia coli* BL21 and the expression vector pET-28a were purchased from Sangon Biotech (Shanghai) Co., Ltd. The experimental methods in the following examples, if not specified, are performed under conventional conditions, such as those described in *Molecular Cloning: A Laboratory Manual,* or as recommended by the respective biological reagent manufacturers. The PCR amplification reaction can be a conventional PCR amplification procedure. *Saccharomyces cerevisiae* is deposited in the American Type Culture Collection (ATCC) with the accession number CICC1964.

For the sake of clear understanding, the present invention has been described quite fully with explanations and examples. It will be clear to those skilled in the art that any equivalent aspects or modifications can be implemented. Therefore, this description and examples should not be interpreted as limiting the scope of the present invention.

High-Performance Liquid Chromatography (HPLC) Detection:
Instrument: 126-Quadrupole Time-of-Flight High-Resolution Mass Spectrometer (Liquid Chromatography-Mass Spectrometry).
HPLC Detection Conditions: Chromatographic column: Asahipak NH2P-504E 4.6 mm×250 mm, 5 µm. Mobile phase: Acetonitrile: Water=65:35. Flow rate: 1 mL/min. Detector: UV-VIS detector. Wavelength: 210 nm. Column temperature: 30°C. Sample volume: 10 µL. Run for 30 min.
Liquid Chromatography-Mass Spectrometry (LC-MS) Detection: H-ESI mode, molecular weight scanning range 400~900.

Nuclear Magnetic Resonance (NMR) Analysis:
Instrument: Agilent DD2-600 Spectrometer.
Detection Method: At room temperature, ¹H-NMR spectrum (600 MHz); ¹³C-NMR spectrum (150 MHz). Chemical shifts are expressed in parts per million (ppm), starting from tetramethylsilane as an internal reference in D₂O (0 ppm). Chemical shifts and coupling constants are calculated from the primary analysis of the spectra.

The LNTII standard was purchased from ELICITYL, France, with the aforementioned HPLC conditions, and its peak time (retention time, rt) was 12.10 min.

### Example 1. The construction of ΔLAC4 marker knockout cassettes for KL (Kluyveromyces lactis) and/or KM (Kluyveromyces marxianus), as well as expression cassettes for enzyme genes of the glycoside hydrolase family 28 in KL and/or KM.

1. Construction of marker KL-ΔLAC4 and/or KM-ΔLAC4 knockout cassettes:

### 1.1 Primer design as shown in Table 1:

**Table 1**

| Primer Name | Sequence |
|---|---|
| LAC4Tef-Kpn1 | CGGGGTACCGAATTTTATACTTAGATAAGTATGTACTTA |
| LAC4Tef-Bamh1 | CGCGGATCCCCTATGCTCCAACGTATACA |
| LAC4Pro-Nde1 | GGAATTCCATATGTATCACGTTGACTTGGTTTAAC |
| LAC4Pro-Not1 | AGAATGCGGCCGCATCTTTCAGTTCTCGATGAGT |
| PUG6-Bamh1 | CGCGGATCCCGATAAGCCAGGTTAACCTG |
| PUG6-Kpn1 | CGGGGTACCGTGGATCTGATATCACCTAATAAC |

### 1.2 PCR amplification

Using the genome of *Kluyveromyces lactis* or *Kluyveromyces maxianus* as a template and LAC4Pro-Nde1 and LAC4Pro-Not1 as primers, the LAC4 promoter region was amplified by PCR, and the LAC4 terminator sequence region was amplified by PCR with primers LAC4Tef-Kpn1 and LAC4Tef-Bamh1. Using the DNA of the recombinant plasmid pUG6, which contains the geneticin resistance gene KanMX4 (nucleotide sequence as shown in SEQ ID NO:9), as a template, the DNA fragment of the resistance gene KanMX4 was amplified by PCR. After verifying the correct size of the bands through agarose gel electrophoresis, the bands were cut out wherein the gene fragments were recovered using the OMEGA gel extraction kit.

The components for PCR amplification are as follows: Phanta Super-Fidelity DNA Polymerase 1 µL, 2× Phanta Max Buffer 25 µL, dNTPs (10 mM) 1 µL, Forward primer (F) 2 µL, Reverse primer (R) 2 µL, Template DNA 1 µL, ddH₂O 18 µL, for a total volume of 50 µL.

PCR amplification steps: (1) Initial denaturation at 95°C for 3 min. (2) Denaturation at 95°C for 0.25 min. (3) Annealing at 54°C for 0.5 min. (4) Extension at 72°C for 0.5 min. Repeat steps (2) to (4) for 30 cycles. (5) Final extension at 72°C for 5 min. (6) Stored at 4°C.

Using the pUG6 plasmid as a template and PUG6-Kpn1 and PUG6-Bamh1 as primers (since the pUG6 plasmid does not have Kpn1 and Bamh1 restriction sites, they need to be introduced via PCR amplification), PCR amplification was performed.

The components for PCR amplification are as follows: Phanta Super-Fidelity DNA Polymerase 1 µL, 2× Phanta Max Buffer 25 µL, dNTPs (10 mM) 1 µL, Forward primer (F) 2 µL, Reverse primer (R) 2 µL, Template DNA 1 µL, ddH₂O 18 µL, for a total volume of 50 µL.

PCR amplification steps: (1) Initial denaturation at 95°C for 3 min. (2) Denaturation at 95°C for 0.25 min. (3) Annealing at 54°C for 0.5 min. (4) Extension at 72°C for 3 min. Repeat steps (2) to (4) for 30 cycles. (5) Final extension at 72°C for 5 min. (6) Stored at 4°C.

After the PCR reaction was completed, agarose gel electrophoresis was performed for identification. Bands of the expected size were then purified and recovered using a gel recovery kit.

### 1.3 Construction of marker KL-ΔLAC4 and KM-ΔLAC4 knockout cassettes

The LAC4 promoter region and terminator sequence region were sequentially inserted into the pUG6 vector by the method of double-enzyme digestion and ligation, constructing KL-ΔLAC4 and KM-ΔLAC4 knockout cassettes.

First restriction-ligation reaction: the terminator sequence obtained in step 1.2 and the pUG6 vector were double digested for approximately 2 hours at 37°C with Kpn1 and Bamh1 respectively. Digestion system: DNA fragment X µL (total DNA approximately 2-3 ng), Kpn1 2 µL, Bamh1 2 µL, Buffer 5 µL, ddH₂O 41-X µL, total volume 50 µL.

After digestion, agarose gel electrophoresis was performed for identification, and bands of the expected size were purified and recovered using a gel recovery kit. The purified and recovered fragments were ligated using T4 DNA ligase at 22°C for approximately 30 minutes. Ligation system: DNA fragment X µL (molar ratio of terminator sequence to pUG6 vector was 3:1), Buffer 1 µL, ddH₂O 9-X µL, total volume 10 µL.

After ligation, *Escherichia coli* was transformed promptly. Correct transformants were picked for plasmid extraction, and the plasmid was used as a new vector for the second restriction-ligation reaction.

Second restriction-ligation reaction: the pUG6 vector with the inserted terminator sequence (already containing Nde1 and Not1 restriction sites) and the promoter region were double digested with Nde1 and Not1 respectively at 37°C for approximately 2 hours. After digestion, agarose gel electrophoresis was performed for identification, and bands of the expected size were purified and recovered using a gel recovery kit. The purified and recovered fragments were ligated using T4 DNA ligase. After ligation, Escherichia coli was transformed promptly. The correct transformants were picked for plasmid extraction to obtain the knockout vector carrying the ΔLAC4 knockout cassette.

2. Construction of *lgtA* KL and *lgtA* KM expression cassettes containing the gene encoding β-N-acetylglucosaminyl transferase:
The *lgtA* KL expression cassettes include KL-ΔLAC4::NMLgtA, KL-ΔLAC4::HPLgtA, KL-ΔLAC4::SELgtA, KL-18S::NMLgtA, KL-18S::HPLgtA, KL-18S::SELgtA.

The *lgtA* KM expression cassettes include KM-ΔLAC4::NMLgtA, KM-ΔLAC4::HPLgtA, KM-ΔLAC4::SELgtA, KM-18S::NMLgtA, KM-18S::HPLgtA, KM-18S::SELgtA.

2.1 Obtaining the fusion sequences of NMLgtA/SELgtA/HPLgtA coding region, ADH1 terminator region, and β-galactosidase LAC4 promoter region:
The full genes NMLgtA, HPLgtA, and SELgtA were synthesized, with their amino acid sequences shown in SEQ ID NO:1, SEQ ID NO:2, and SEQ ID NO:3, respectively.

Promoter region sequences: as shown in SEQ ID NO:6, derived from the LAC4 promoter region of *Kluyveromyces lactis*; as shown in SEQ ID NO:7, derived from the LAC4 promoter region of *Kluyveromyces marxianus.*

ADH1 terminator region sequence, as shown in SEQ ID NO:8, derived from *Saccharomyces cerevisiae.*

PCR amplification was performed using the NMLgtA/HPLgtA/SELgtA genes, *Kluyveromyces lactis* genome, *Kluyveromyces marxianus* genome, and *Saccharomyces cerevisiae* genome as templates respectively.

PCR amplification components: Phanta Super-Fidelity DNA Polymerase 1 µL, 2×Phanta Max Buffer 25 µL, dNTPs (10 mM) 1 µL, Forward primer (F) 2 µL, Reverse primer (R) 2 µL, Template DNA 1µL, ddH₂O 18 µL, for a total volume of 50 µL.

PCR Amplification Steps: (1) Initial denaturation at 95°C for 3 min. (2) Denaturation at 95°C for 0.25 min. (3) Annealing at 54°C for 0.5 min. (4) Extension at 72°C for 1.5 min. Repeat steps (2) to (4) for 30 cycles. (5) Final extension at 72°C for 5 min. (6) Stored at 4°C.

After the PCR reaction was completed, agarose gel electrophoresis was performed for identification. Bands of the expected size were then purified and recovered using a gel recovery kit. The DNA concentration of recovered products was measured using a microvolume spectrophotometer and then the products were diluted to a concentration of 1 ng/µL with ddH₂O. Fusion PCR amplification was performed using the diluted DNA fragments as templates, with ADH1-Not1 and LAC4Pro-Nde1/LAC4Pro-S as primers.

The components for PCR amplification are as follows: Phanta Super-Fidelity DNA Polymerase 1 µL, 2× Phanta Max Buffer 25 µL, dNTPs (10 mM) 1 µL, Forward primer (F) 2 µL, Reverse primer (R) 2 µL, Template DNA 1 µL, ddH₂O 18 µL, for a total volume of 50 µL.

### PCR amplification

Steps: (1) Initial denaturation at 95°C for 3 min. (2) Denaturation at 95°C for 0.25 min. (3) Annealing at 54°C for 0.5 min. (4) Extension at 72°C for 2.5 min. Repeat steps (2) to (4) for 30 cycles. (5) Final extension at 72°C for 5 min. (6) Stored at 4°C.

After the PCR reaction was completed, agarose gel electrophoresis was performed for identification. Bands of the expected size were then purified and recovered using a gel recovery kit to obtain the fusion sequences of NMLgtA/SELgtA/HPLgtA coding region, ADH1 terminator region, and β-galactosidase LAC4 promoter region.

2.2 Promoter region, terminator sequence region, pUG6 vector sequences, and pUG6 vector resistance sequences were obtained by PCR amplification.

PCR amplification was performed using the genomes of *Kluyveromyces lactis, Kluyveromyces marxianus,* and the pUG6 vector as templates.

The components for PCR amplification are as follows: Phanta Super-Fidelity DNA Polymerase 1 µL, 2× Phanta Max Buffer 25 µL, dNTPs (10 mM) 1 µL, Forward primer (F) 2 µL, Reverse primer (R) 2 µL, Template DNA 1 µL, ddH₂O 18 µL, for a total volume of 50 µL.

### PCR amplification

Steps: (1) Initial denaturation at 95°C for 3 min. (2) Denaturation at 95°C for 0.25 min. (3) Annealing at 54°C for 0.5 min. (4) Extension at 72°C for 1.5 min. Repeat steps (2) to (4) for 30 cycles. (5) Final extension at 72°C for 5 min. (6) Stored at 4°C.

After the PCR reaction was completed, agarose gel electrophoresis was performed for identification. Bands of the expected size were then purified and recovered using a gel recovery kit.

### 2.3 Construction of expression cassettes

The fusion sequences obtained in step 2.1, along with the ΔLAC4 knockout vector were double digested with Nde1 and Not1 at 37°C for about 2 hours. After digestion, agarose gel electrophoresis was conducted for identification, and bands of the expected size were purified and recovered using a gel recovery kit. The purified and recovered fragments were ligated using T4 DNA ligase. After ligation, immediate transformation into *Escherichia coli* was carried out. The correct transformants were selected for plasmid extraction, obtaining expression vectors KL-ΔLAC4::NMLgtA, KL-ΔLAC4::SELgtA, KL-ΔLAC4::HPLgtA, KM-ΔLAC4::NMLgtA, KM-ΔLAC4::SELgtA, and KM-ΔLAC4::HPLgtA, each respectively carrying NMLgtA, SELgtA, and HPLgtA expression cassettes.

The fusion sequences obtained in step 2.1 were mixed with upstream and downstream homologous arm sequences, backbone sequences, and resistance sequences from step 2.2 in a total volume of 5 µL at a molar ratio of 1:1:1:1:1, then 5 µL of seamless cloning MIX was added for ligation at 50°C for 30-60 minutes. After ligation, immediate transformation into *Escherichia coli* was carried out, and the correct transformants were selected for plasmid extraction, obtaining expression vectors KL-18S::NMLgtA, KL-18S::SELgtA, KL-18S::HPLgtA, KM-18S::NMLgtA, KM-18S::SELgtA, and KM-18S::HPLgtA, each respectively carrying NMLgtA, SELgtA, and HPLgtA expression cassettes (the methods of restriction digestion, ligation, and *Escherichia coli* transformation were the same as in Example 1, section 1.3).

The sources of reagents and kits used in this example were shown in Table 2.

**Table 2**

| Reagent/Kit | Source | Reagent/Kit | Source |
|---|---|---|---|
| Gel Recovery Kit | OMEGA | Peptone | OXOID |
| Plasmid Extraction Kit | OMEGA | Glucose | Sinopharm Group |
| High Fidelity Enzyme | Vazyme | Quartz Sand | Sinopharm Group |
| DNA maker | RuiBiotech | T4 DNA Ligase | NEB |
| Agarose | Tsingke Biotech | Tris | Sinopharm Group |
| DNA Extraction Solution | Dingguo Changsheng Biotech | Na₂EDTA·H₂O | Sinopharm Group |
| Yeast Powder | OXOID | SDS | Sinopharm Group |

The solvents and buffer formulations used in this example are as follows:
50×TAE solution: prepared with ddH₂O, containing 2 M Tris, 100 mM Na₂EDTA·H₂O, 2% SDS, adjusted to pH 8.5.
Genomic DNA extraction buffer: prepared with ddH₂O, containing 200 mM Tris-HCl, 250 mM NaCl, 2% SDS, 25 mM EDTA, pH adjusted to 8.0.
*Escherichia coli* culture medium: composed of 10 g/L peptone, 5 g/L yeast extract, 10 g/L sodium chloride, and water. For solid media, an additional 20 g/L of agar powder was added. The medium was autoclaved at 121°C for 20 minutes before use.
Yeast culture medium: composed of 20 g/L peptone, 10 g/L yeast extract, 20 g/L glucose, and water. For solid media, an additional 20 g/L of agar powder was added. The medium was autoclaved at 115°C for 30 minutes before use.

Cultivation conditions for this example:
*Escherichia coli* was cultured on solid plates in an incubator set at 37°C and in shaking flasks at 37°C with a speed of 200 rpm.

Yeast was cultured on solid plates in an incubator set at 30°C and in shaking flasks at 30°C with a speed of 200 rpm.

Yeast genomic DNA extraction method of this example:
(1) A single colony of *Kluyveromyces lactis* was inoculated into 1 mL YPD medium in a 10 mL centrifuge tube and cultured overnight at 30°C, 200 rpm.
(2) 600 µL of culture was centrifuged in a 1.5 mL EP tube at 10,000 rpm for 1 minute, and the supernatant was discarded.
(3) 600-800 µL of genomic DNA extraction buffer and 100 µL of quartz sand were added, followed by vigorous shaking for 5 minutes.
(4) The mixture was incubated in a 65°C water bath for 30 minutes, inverting every 10 minutes.
(5) The supernatant was transferred to a new 1.5 mL EP tube, mixed with an equal volume of DNA extraction solution, and pipette mixed.
(6) The mixture was centrifuged at 13,000 rpm for 10 minutes. 400 µL of the supernatant was transferred to a new 1.5 mL EP tube.
(7) 0.6 volume of isopropanol and 40 µL of 3M sodium acetate were added, mixed, and incubated at -20°C for 30 minutes.
(8) The mixture was centrifuged at 13,000 rpm for 10 minutes, the supernatant was discarded, and the yeast genomic DNA was obtained.
(9) The DNA was washed twice with 70% ethanol, air-dried, and dissolved in ddH₂O, and stored at -20°C.

### Example 2: Construction of recombinant strains of Kluyveromyces lactis and Kluyveromyces marxianus

1. After the successful construction of the ΔLAC4 knockout cassette, yeast transformation was performed, resulting in control strains StrainKL-1 and StrainKM-1. The method for yeast transformation is described in this example.
2. After the successful construction of the KL-ΔLAC4::NMLgtA, KL-ΔLAC4::SELgtA, KL-ΔLAC4::HPLgtA, KL-18S::NMLgtA, KL-18 S:: SELgtA, and KL-18S::HPLgtA expression cassettes, yeast transformation was performed for each cassette, resulting in recombinant strains StrainKL-2-01, StrainKL-2-05, StrainKL-2-02 and StrainKL-2-03, StrainKL-2-05, and StrainKL-2-04.

After the successful construction of the KM-ΔLAC4::NMLgtA, KM-ΔLAC4::SELgtA, KM-ΔLAC4::HPLgtA, KM-18S::NMLgtA, KM-18S::SELgtA, and KM-18S::HPLgtA expression cassettes, yeast transformation was performed for each cassette, resulting in recombinant strains StrainKM-2-01, StrainKM-2-05, StrainKM-2-02, StrainKM-2-03, StrainKM-2-06 and StrainKM-2-04.

The yeast transformation method was as follows:
(1) Preparation of yeast competent cells: a small amount of yeast strain from the frozen stock was streaked on solid medium plates and incubated upside down at 30°C for 2 days. A single yeast colony was then inoculated into 50 mL of liquid medium and cultured at 30°C, 220 rpm until the OD₆₀₀ reached 0.8-1.5. The cells were collected, washed with 25 mL of sterile water, and centrifuged at 1500 ×g for 10 minutes at room temperature. The supernatant was discarded, and the cells were resuspended in 1 mL of 100 mM lithium chloride buffer, centrifuged at 12,000 rpm for 30 seconds, and the supernatant was discarded again. The cells were then resuspended in 400 µL of 100 mM lithium chloride buffer to obtain competent yeast cells, which were aliquoted into 50 µL per tube for transformation.
(2) Transformation: the prepared competent yeast cells were centrifuged, and the residual lithium chloride solution was removed using Tips. For each transformation, the following were added in order: 50% PEG3350 (240 µL), 1 M LiCl (36 µL), 2 mg/mL single-stranded salmon sperm DNA (25 µL), and 5-10 µg of plasmid DNA in 50 µL H₂O (50 µL). The mixture was vigorously vortexed until the cell pellet was completely dispersed, then incubated in a 30°C water bath for 30 minutes and subjected to heat shock at 42°C for 20-25 minutes. After centrifugation at 8000 rpm for 10 minutes, the yeast cells were collected, resuspended in 500 µL of liquid medium, and incubated on a shaker at 30°C. After 1-4 hours, 25-100 µL of culture was spread onto selective medium plates and incubated upside down at 30°C.

The competent yeast cells mentioned are those of the original *Kluyveromyces lactis* strain StrainKL-0 and the original *Kluyveromyces marxianus* strain StrainKM-0. The expression cassettes KL-ΔLAC4::NMLgtA, KL-ΔLAC4:: SELgtA, and KL-ΔLAC4::HPLgtA were introduced into the competent cells of original *Kluyveromyces lactis* strain StrainKL-0. The expression cassettes KM-18S::NMLgtA, KM-18S::SELgtA, and KM-18S::HPLgtA were introduced into the competent cells of the original *Kluyveromyces marxianus* strain StrainKM-0.

The plasmid DNA used was the KL-ΔLAC4 knockout cassette constructed in Example 1, KL-ΔLAC4::NMLgtA, KL-ΔLAC4::SELgtA, KL-ΔLAC4::HPLgtA, and expression cassettes KL-18S::NMLgtA, KL-18S::SELgtA, and KL-18S::HPLgtA.

The plasmid DNA used was the KM-ΔLAC4 knockout cassette constructed in Example 1, KM-ΔLAC4::NMLgtA, KM-ΔLAC4::SELgtA and KM-ΔLAC4::HPLgtA, and expression cassettes KM-18S::NMLgtA, KM-18S::SELgtA and KM-18S::HPLgtA.

3. Verification of recombinant strains of *Kluyveromyces lactis* and *Kluyveromyces marxianus:*
After 2-3 days of static incubation on plates, the grown transformant colonies were verified. The verification method was as follows:
A single transformant colony was picked and inoculated into 1.5 mL of liquid medium 1, then shaken cultured overnight at 30°C, 200 rpm. Subsequently, 50 µL of the culture was transferred into 1.5 mL of liquid medium 1 and liquid medium 2, and shaken cultured overnight at 30°C, 200 rpm. The growth was observed, and transformants that grew normally on solid medium 1 but hardly grew on solid medium 2 were selected for preservation as recombinant *Kluyveromyces lactis* strains StrainKL-2-01, StrainKL-2-02, StrainKL-2-03, StrainKL-2-04, StrainKL-2-05, StrainKL-2-06, and recombinant *Kluyveromyces marxianus* strains StrainKM-2-01, StrainKM-2-02, StrainKM-2-03, StrainKM-2-04, StrainKM-2-05, StrainKM-2-06. The correspondence between these strains and the expression cassettes was shown in Table 3 and 4.

**Table 3. Correspondence between recombinant Kluyveromyces lactis strains and expression cassettes**

| Expression Cassette | Recombinant Strain |
|---|---|
| KL-ΔLAC4::NMLgtA | StrainKL-2-01 |
| KL-ΔLAC4::HPLgtA | StrainKL-2-02 |
| KL-18S::NMLgtA | StrainKL-2-03 |
| KL-18S::HPLgtA | StrainKL-2-04 |
| KL-ΔLAC4::SELgtA | StrainKL-2-05 |
| KL-18S::SELgtA | StrainKL-2-06 |

**Table 4. Correspondence between recombinant Kluyveromyces marxianus strains and expression cassettes**

| Expression Cassette | Recombinant Strain |
|---|---|
| KM-ΔLAC4::NMLgtA | StrainKM-2-01 |
| KM-ΔLAC4::HPLgtA | StrainKM-2-02 |
| KM-18S::NMLgtA | StrainKM-2-03 |
| KM-18S::HPLgt A | StrainKM-2-04 |
| KM-ΔLAC4::SELgtA | StrainKM-2-05 |
| KM-18S::SELgtA | StrainKM-2-06 |

The specific conditions of the media used in this example are as follows:
Solid medium 1: composed of 20 g/L peptone, 10 g/L yeast extract, 20 g/L glucose, and 20 g/L agar powder in water, sterilized at 115°C under high-pressure steam for 30 minutes before use.
Liquid medium 1: composed of 20 g/L peptone, 10 g/L yeast extract, and 20 g/L glucose in water, sterilized at 115°C under high-pressure steam for 30 minutes before use.
Solid medium 2: composed of 20 g/L peptone, 10 g/L yeast extract, 20 g/L lactose, and 20 g/L agar powder in water, sterilized at 115°C under high-pressure steam for 30 minutes before use.
Liquid medium 2: composed of 20 g/L peptone, 10 g/L yeast extract, and 20 g/L lactose in water, sterilized at 115°C under high-pressure steam for 30 minutes before use.

### Example 3: Fermentation Experiments with Kluyveromyces lactis and Kluyveromyces marxianus Strains along with their respective control strains.

The original strains of *Kluyveromyces lactis* and *Kluyveromyces marxianus,* along with their respective control strains, were subjected to a two-stage cultivation process for the synthesis verification of LNTII.

In the first stage, the cell growth phase, the strains were cultured with glucose as the carbon source for the accumulation of biomass and enzymes until the cell growth reached the late logarithmic or stationary phase. The strains were streaked on solid medium and incubated at 30°C for 2-3 days. A single colony was then inoculated into 1.5 mL of liquid medium and shaken overnight at 30°C, 200 rpm. Subsequently, the culture was inoculated at a 2% inoculum into a 50 mL liquid medium in a shaking flask and cultured at 30°C, 200 rpm until OD₆₀₀ reached 1. This culture was then inoculated at a 2% inoculum into 5 L of liquid medium in a 10 L volume fermenter and shaken cultured at 30°C, 200 rpm for biomass accumulation.

In the second stage, the product synthesis phase, the culture was shaken at 30°C, 200 rpm for up to 40 hours before starting a continuous feeding process (the amount of feed added was recorded), with a total fermentation duration of 72 hours. After 72 hours of fermentation, the fermentation broth was centrifuged, the cells were disrupted using a high-pressure homogenizer, proteins were removed by centrifugation, and the supernatant was filtered through a 0.22 µm filter for impurity removal. The LNTII contents in the supernatants were determined using HPLC, and the ethanol contents in the fermentation broths of the original strains of *Kluyveromyces lactis* (StrainKL-0) and *Kluyveromyces marxianus* were measured using a biosensor. The results were recorded in Table 5.

**Table 5. Analysis of the fermentation broths of Kluyveromyces lactis and Kluyveromyces marxianus original strains**

| Strain | LNTII Production, g/L | Ethanol Production, g/L |
|---|---|---|
| StrainKL-0 | 0 | 24.3 |
| StrainKL-1 | 0 | 22.1 |
| StrainKM-0 | 0 | 25.1 |
| StrainKM-1 | 0 | 21.5 |

This indicates that the original and control strains of *Kluyveromyces lactis* and *Kluyveromyces marxianus* did not have the capability to produce LNTII.

The specific media used in this example were as follows:
Solid medium: composed of 20 g/L peptone, 10 g/L yeast extract, 20 g/L glucose, and 20 g/L agar powder, sterilized with high-temperature steam at 115°C for 30 minutes.
Liquid medium: composed of 20 g/L peptone, 10 g/L yeast extract, 20 g/L glucose, 8 g/L lactose, and final concentrations of 5 mM each of dipotassium hydrogen phosphate, magnesium sulfate, ammonium sulfate, and manganese sulfate, sterilized with high-temperature steam at 115°C for 30 minutes.
Supplement: composed of 2 g/L lactose, 20 g/L glucose, and final concentrations of 5 mM each of dipotassium hydrogen phosphate, magnesium sulfate, ammonium sulfate, and manganese sulfate.

### Example 4: Synthesis of LNTII catalyzed by recombinant Kluyveromyces lactis (StrainKL-2-01) and recombinant Kluyveromyces marxianus (StrainKM-2-01).

The recombinant strains StrainKL-2-01 and/or StrainKM-2-01 were also subjected to a two-stage cultivation process for the preparation of LNTII.

In the first phase, the growth phase, the cells were cultured using glucose as the carbon source for the accumulation of biomass and enzymes until they reached the late logarithmic or stationary phase. The strains were streaked on solid medium and, after incubating at 30°C for 2-3 days, a single colony was inoculated into 1.5mL of liquid medium and cultured at 30°C, 200 rpm until OD₆₀₀ reached 1. This culture was then inoculated at a 2% (v/v) volume into 5L of liquid medium in a 10L fermenter and shaken overnight at 30°C, 200 rpm. Subsequently, it was inoculated into a 50 mL liquid medium at a 2% (v/v) volume and cultured at 30°C, 200 rpm for biomass accumulation.

In the second phase, the product synthesis phase, was the same as the second phase of Example 3, except for the recombinant strains.

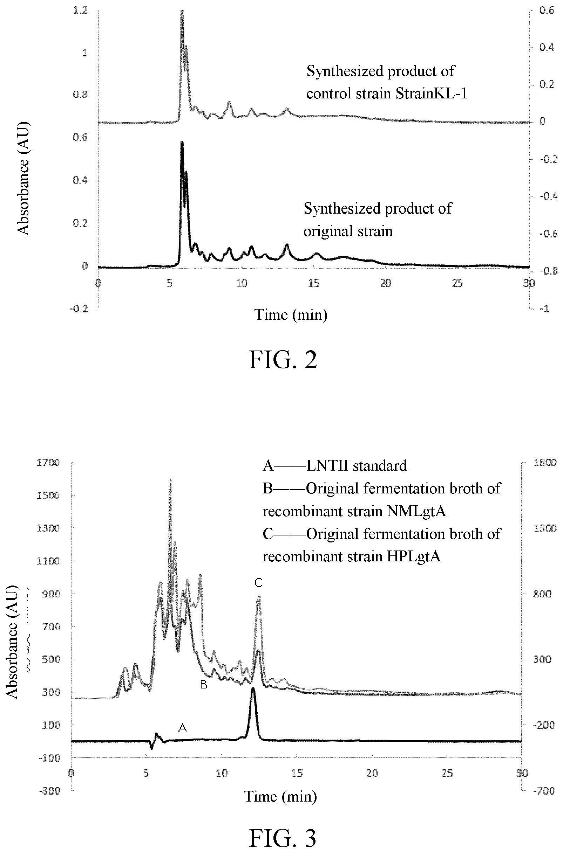

The LNTII content in the fermentation broth of StrainKL-2-01 and StrainKM-2-01 was measured by HPLC as 8.53 g/L and 8.75 g/L respectively, with lactose to LNTII conversion rates of 51.53% and 53.53%. The HPLC profile of the original fermentation broth of StrainKL-2-01 was shown in FIG. 2, curve B, and the HPLC profile of the purified fermentation broth was shown in FIG. 3.

The HPLC profile of the product synthesized by recombinant strain StrainKM-2-01 was identical to that of StrainKL-2-01.

Additionally, the ethanol contents in the fermentation broths of StrainKL-2-01 and StrainKM-2-01 were measured using a biosensor, yielding approximately 25.1 g/L and 25.7 g/L, respectively.

The solid media, liquid media, and supplements in this example were the same as in Example 3.

### Example 5: Synthesis of LNTII catalyzed by recombinant Kluyveromyces lactis (StrainKL-2-03) and recombinant Kluyveromyces marxianus (StrainKM-2-03)

The recombinant strains StrainKL-2-03 and StrainKM-2-03 were also subjected to a two-stage cultivation process for the preparation of LNTII.

The first stage, the growth phase, was the same as the first stage of Example 4, except for the strains.

In the second stage, the product synthesis phase, except for the recombinant strains, all other procedures were the same as in the second stage of Example 4.

The LNTII contents in the fermentation broths of StrainKL-2-03 and StrainKM-2-03 were determined by HPLC to be 7.21 g/L and 7.15 g/L, respectively, with lactose to LNTII conversion rates of 45.24% and 44.21%. Additionally, the ethanol content was measured using a biosensor, yielding approximately 25 g/L and 24.3 g/L, respectively.

The solid media, liquid media, and supplements in this example were the same as in Example 3.

### Example 6: Synthesis of LNTII catalyzed by recombinant Kluyveromyces lactis (StrainKL-2-02) and recombinant Kluyveromyces marxianus (StrainKM-2-02)

The recombinant strains StrainKL-2-02 and StrainKM-2-02 were also subjected to a two-stage cultivation process for the preparation of LNTII.

The first stage, the growth phase, was the same as the first stage of Example 4, except for the strains.

In the second stage, the product synthesis phase, except for the recombinant strains, all other procedures were the same as in the second stage of Example 4.

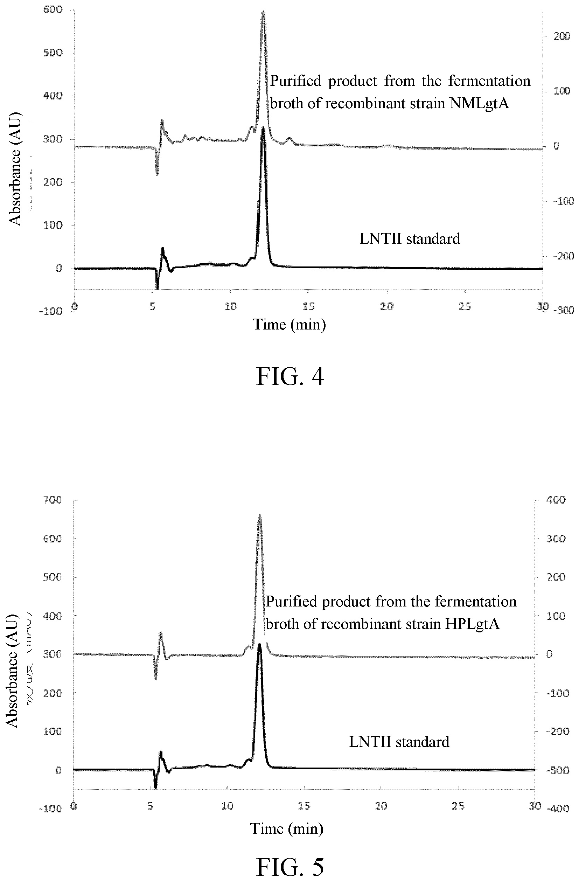

The LNTII contents in the fermentation broths of StrainKL-2-02 and StrainKM-2-02 were measured by HPLC as 15.23 g/L and 15.27 g/L respectively, with lactose to LNTII conversion rates of 95.57% and 95.71%. The HPLC profile of the original fermentation broth of recombinant strain StrainKL-2-02 was shown in FIG. 2, curve C, and the HPLC profile of the purified fermentation broth was shown in FIG. 4.

The HPLC profile of the product synthesized by recombinant strain StrainKM-2-02 was identical to that of StrainKL-2-02.

Additionally, the ethanol contents in the fermentation broths of StrainKL-2-02 and StrainKM-2-02 were measured using a biosensor, yielding approximately 25.2 g/L and 25.6 g/L, respectively.

The solid media, liquid media, and supplements in this example were the same as in Example 3.

### Example 7: Synthesis of LNTII catalyzed by recombinant Kluyveromyces lactis (StrainKL-2-04) and recombinant Kluyveromyces marxianus (StrainKM-2-04)

The recombinant strains StrainKL-2-04 and StrainKM-2-04 were also subjected to a two-stage cultivation process for the preparation of LNTII.

The first stage, the growth phase, was the same as the first stage of Example 4, except for the strains.

In the second stage, the product synthesis phase, except for the recombinant strains, all other procedures were the same as in the second stage of Example 4.

The LNTII contents in the fermentation broths of StrainKL-2-04 and StrainKM-2-04 were determined by HPLC to be 14.57 g/L and 14.71 g/L, respectively, with lactose to LNTII conversion rates of 91.42% and 92.02%. Additionally, the ethanol content was measured using a biosensor, yielding approximately 24.6 g/L and 24.9 g/L, respectively.

The solid media, liquid media, and supplements in this example were the same as in Example 3.

### Example 8: Synthesis of LNTII catalyzed by recombinant Kluyveromyces lactis (StrainKL-2-05) and recombinant Kluyveromyces marxianus (StrainKM-2-05)

The recombinant strains StrainKL-2-05 and StrainKM-2-05 were also subjected to a two-stage cultivation process for the preparation of LNTII.

The first stage, the growth phase, was the same as the first stage of Example 4, except for the strains.

In the second stage, the product synthesis phase, except for the recombinant strains, all other procedures were the same as in the second stage of Example 4.

The LNTII contents in the fermentation broths of StrainKL-2-05 and StrainKM-2-05 were determined by HPLC to be 3.23 g/L and 3.27 g/L, respectively, with lactose to LNTII conversion rates of 20.27% and 20.52%.

The HPLC profile of the product synthesized by recombinant strain StrainKM-2-05 was identical to that of StrainKL-2-05.

Additionally, the ethanol contents in the fermentation broths of StrainKL-2-05 and StrainKM-2-05 were measured using a biosensor, yielding approximately 26.1 g/L and 26.3 g/L, respectively.

The solid media, liquid media, and supplements in this example were the same as in Example 3.

### Example 9: Synthesis of LNTII catalyzed by recombinant Kluyveromyces lactis (StrainKL-2-06) and recombinant Kluyveromyces marxianus (StrainKM-2-06)

The recombinant strains StrainKL-2-06 and StrainKM-2-06 were also subjected to a two-stage cultivation process for the preparation of LNTII.

The first stage, the growth phase, was the same as the first stage of Example 4, except for the strains.

In the second stage, the product synthesis phase, except for the recombinant strains, all other procedures were the same as in the second stage of Example 4

The LNTII contents in the fermentation broths of StrainKL-2-06 and StrainKM-2-06 were determined by HPLC to be 2.45 g/L and 2.17 g/L, respectively, with lactose to LNTII conversion rates of 15.37% and 13.61%. Additionally, the ethanol contents were measured using a biosensor, yielding approximately 26.3 g/L and 27.1 g/L, respectively.

The solid media, liquid media, and supplements in this example were the same as in Example 3.

### Example 10: Synthesis of LNTII catalyzed by recombinant Kluyveromyces lactis (StrainKL-2-01) and recombinant Kluyveromyces marxianus (StrainKM-2-01)

The first stage, the growth phase, was the same as the first stage of Example 4, except for the strains.

In the second stage, the product synthesis phase, the culture was shaken at 15°C, 200 rpm for up to 40 hours before starting a continuous feeding process, with a total fermentation duration of 72 hours. After 72 hours of fermentation, the fermentation broth was centrifuged, the cells were disrupted using a high-pressure homogenizer, proteins were removed by centrifugation, and the supernatant was filtered through a 0.22 µm filter for impurity removal. The LNTII content in the supernatant was determined using HPLC.

The LNTII contents in the fermentation broths of StrainKL-2-01 and StrainKM-2-01 were determined by HPLC to be 4.73 g/L and 4.67 g/L, respectively, with lactose to LNTII conversion rates of 29.81% and 29.68%. Additionally, the ethanol content was measured using a biosensor, yielding approximately 21.4 g/L and 21.5 g/L, respectively.

The specific media used in this example were as follows:
The solid medium used in this example was the same as in Example 3.

The liquid medium was composed of 20 g/L peptone, 10 g/L yeast extract, 20 g/L glucose, and 8 g/L lactose, with a final concentration of 3 mM each of ammonium sulfate and manganese sulfate. This medium was sterilized at 115°C under high-pressure steam for 30 minutes before use.

The supplement consisted of 2 g/L lactose, 20 g/L glucose, with final concentrations of 3 mM each of ammonium sulfate and manganese sulfate.

### Example 11: Synthesis of LNTII catalyzed by recombinant Kluyveromyces lactis (StrainKL-2-01) and recombinant Kluyveromyces marxianus (StrainKM-2-01)

The first stage, the growth phase, was the same as the first stage of Example 4, except for the strains.

In the second stage, the product synthesis phase, the culture was shaken at 60°C, 200 rpm for up to 40 hours before starting a continuous feeding process, with a total fermentation duration of 72 hours. After 72 hours of fermentation, the fermentation broth was centrifuged, the cells were disrupted using a high-pressure homogenizer, proteins were removed by centrifugation, and the supernatant was filtered through a 0.22 µm filter for impurity removal. The LNTII content in the supernatant was determined using HPLC.

The LNTII contents in the fermentation broths of StrainKL-2-01 and StrainKM-2-01 were determined by HPLC to be 1.12 g/L and 1.15 g/L, respectively, with lactose to LNTII conversion rates of 7.02% and 7.07%. Additionally, the ethanol contents were measured using a biosensor, yielding approximately 18.0 g/L and 18.2 g/L, respectively.

The specific media used in this example were as follows:
The solid medium used in this example was the same as in Example 3.

The liquid medium was prepared with 20 g/L peptone, 10 g/L yeast extract, 20 g/L glucose, and 8 g/L lactose, with final concentrations of 7 mM dipotassium hydrogen phosphate and magnesium sulfate. This medium was then sterilized at 115°C under high-pressure steam for 30 minutes before use.

The supplement consisted of 2 g/L lactose, 20 g/L glucose, with final concentrations of 7 mM dipotassium hydrogen phosphate and magnesium sulfate.

### Example 12: Synthesis of LNTII catalyzed by recombinant Kluyveromyces lactis (StrainKL-2-01) and recombinant Kluyveromyces marxianus (StrainKM-2-01)

The first stage, the growth phase, was the same as the first stage of Example 4, except for the strains.

In the second stage, the product synthesis phase, the culture was shaken at 28°C, 200 rpm for up to 40 hours before starting a continuous feeding process, with a total fermentation duration of 72 hours. After 72 hours of fermentation, the fermentation broth was centrifuged, the cells were disrupted using a high-pressure homogenizer, proteins were removed by centrifugation, and the supernatant was filtered through a 0.22 µm filter for impurity removal. The LNTII content in the supernatant was determined using HPLC.

The LNTII contents in the fermentation broths of StrainKL-2-01 and StrainKM-2-01 were determined by HPLC to be 7.89 g/L and 7.87 g/L, respectively, with lactose to LNTII conversion rates of 49.51% and 49.49%. Additionally, the ethanol contents were measured using a biosensor, yielding approximately 24.8g/L and 24.7 g/L, respectively.

The specific media used in this example were as follows:
The solid medium used in this example was the same as in Example 3.

The liquid medium was composed of 20 g/L peptone, 10 g/L yeast extract, 20 g/L glucose, and 8 g/L lactose, with a final concentration of 6 mM magnesium sulfate. This medium was sterilized at 115°C under high-pressure steam for 30 minutes before use.

The supplement included 2 g/L lactose, 20 g/L glucose, with a final concentration of 6 mM magnesium sulfate.

### Example 13: Synthesis of LNTII catalyzed by recombinant Kluyveromyces lactis (StrainKL-2-01) and recombinant Kluyveromyces marxianus (StrainKM-2-01)

The first stage, the growth phase, was the same as the first stage of Example 4, except for the strains.

In the second stage, the product synthesis phase, all procedures were the same as in the second stage of Example 10, except for the liquid medium and supplement.

The LNTII contents in the fermentation broths of StrainKL-2-01 and StrainKM-2-01 were determined by HPLC to be 7.17 g/L and 7.15 g/L, respectively, with lactose to LNTII conversion rates of 44.99% and 44.97%. Additionally, the ethanol contents were measured using a biosensor, yielding approximately 24.3g/L and 24.5 g/L, respectively.

The specific media used in this example were as follows:
The solid medium used in this example was the same as in Example 3.

The liquid medium was composed of 20 g/L peptone, 10 g/L yeast extract, 20 g/L glucose, and 8 g/L lactose, with a final concentration of 6 mM potassium sulfate. This medium was sterilized at 115°C under high-pressure steam for 30 minutes before use.

The supplement included 2 g/L lactose, 20 g/L glucose, with a final concentration of 6 mM potassium sulfate.

### Example 14: Purification of the synthesized product from Example 6

The synthesized product from Example 6 was purified using an activated carbon adsorption method, with the following steps:
(1) 100 ml of the fermentation broth from Example 6 was taken, and activated carbon was added at a final volume to activated carbon mass ratio of 1-2%. The mixture was then shaken well.
(2) The mixture was placed on a shaker and agitated at 150-250 rpm at a temperature of 30-40°C for 1-3 hours.
(3) The solution was then freeze-dried at low temperatures to obtain a powder. The purity of the LNTII product synthesized by the recombinant strains StrainKL-2-02 and StrainKM-2-02, as determined by HPLC, was 97.2% and 97.3%, respectively.

### Example 15: Due to the strong randomness of mutations caused by ultraviolet (UV) mutagenesis and the randomness of the effects of the mutations, multiple rounds of UV mutagenesis screening are usually required to obtain effective positive mutations. This process can be labor-intensive and there is a possibility of not obtaining effective positive mutations. However, UV mutagenesis is yet a common method for mutation breeding due to its simple equipment, low cost, and the ability to obtain a large number of mutants in a short time.

The applicants used the recombinant strains StrainKL-2-01, StrainKL-2-02, StrainKM-2-01, and StrainKM-2-02 as starting strains and performed genetic modification of the recombinant strains through UV mutagenesis to further increase the LNTII production of the recombinant strains.

The recombinant strains StrainKL-2-01, StrainKL-2-02, StrainKM-2-01, and StrainKM-2-02 were cultured with glucose as the carbon source for the accumulation of biomass and enzymes until the cell growth reached the late logarithmic or stationary phase. The strains were streaked onto solid medium and incubated at 30°C for 2-3 days. Single colonies were inoculated into 1.5 mL of liquid medium and shaken overnight at 30°C, 200 rpm. The cells were washed with sterile water to prepare a suspension, diluted to 1×10⁶ cells/mL, and exposed to UV light (40W) at a distance of about 22 cm for 2-10 minutes until a mortality rate of over 90% was achieved. The cells were then spread onto plates and incubated at 30°C for 48 hours.

In the first round of UV mutagenesis, approximately 200 mutant colonies of StrainKL-2-01/StrainKM-2-01, and about 215 mutant colonies of StrainKL-2-02/StrainKM-2-02 were obtained. Each colony was inoculated into a 96-well plate containing 200 µL of liquid medium 1 and incubated at 30°C, 200 rpm for 24 hours. After centrifugation to remove the upper layer of the medium, 200 µL of liquid medium 2 was added, and the culture was incubated at 30°C, 200 rpm for 72 hours. Mutant strains with significantly increased LNTII production were screened compared to the starting strains StrainKL-2-01/StrainKL-2-02 and StrainKM-2-01/StrainKM-2-02.

The results showed that none of the mutant strains obtained from the first round of UV mutagenesis had a higher LNTII production in the fermentation supernatant than the starting strains. The applicants continued to carry out eight more rounds of mutation screening using the same method and eventually obtained one mutant strain for each starting strain with a significantly higher LNTII production, named StrainKL-2-07/StrainKL-2-08 and StrainKM-2-07/StrainKM-2-08, respectively.

The selected mutant strains StrainKL-2-07/StrainKL-2-08 and StrainKM-2-07/StrainKM-2-08 were transferred to liquid medium 1, incubated at 30°C, 200 rpm for 24 hours, then centrifuged to remove the upper layer of the medium, and transferred to liquid medium 2. They were incubated at 30°C, 200 rpm for 40 hours before starting the feeding process, with a total fermentation duration of 72 hours. After 72 hours of fermentation, the fermentation broth was centrifuged, the cells were disrupted using a high-pressure homogenizer. The proteins were removed by centrifugation, and the supernatant was filtered through a 0.22 µm filter for impurity removal. The LNTII content was then determined using HPLC. The results were as follows: the concentrations of LNTII in the fermentation broth of StrainKL-2-07/StrainKL-2-08 were 10.32 g/L and 20.44 g/L, respectively, with lactose to LNTII conversion rates of 64.76% and 98.76%. For StrainKM-2-07/StrainKM-2-08, the LNTII yields were 10.81 g/L and 20.67 g/L, respectively, with lactose to LNTII conversion rates of 67.84% and 99.06%, significantly higher than the starting strains.

The solid medium was composed of 20 g/L peptone, 10 g/L yeast extract, 20 g/L glucose, and 20 g/L agar powder. The medium was sterilized at 115°C under high-pressure steam for 30 minutes before use.

The liquid medium 1 was composed of 20 g/L peptone, 10 g/L yeast extract, and 20 g/L glucose in water. The medium was sterilized at 115°C under high-pressure steam for 30 minutes before use.

The liquid medium 2 was composed of 20 g/L peptone, 10 g/L yeast extract, 20 g/L glucose, 8 g/L lactose, and final concentrations of 5 mM each of dipotassium hydrogen phosphate, magnesium sulfate, ammonium sulfate, and manganese sulfate. The medium was sterilized with high-temperature steam at 115°C for 30 minutes.

The supplement was composed of 5 g/L lactose, 20 g/L glucose, and final concentrations of 5 mM each of dipotassium hydrogen phosphate, magnesium sulfate, ammonium sulfate, and manganese sulfate.

### Example 16: Synthesis of LNTII catalyzed by recombinant strains StrainKL-2-01 and/or StrainKL-2-02 with StrainKM-2-01 and/or StrainKM-2-02

The recombinant strains StrainKL-2-01 and/or StrainKL-2-02 with StrainKM-2-01 and/or StrainKM-2-02 were used for the two-stage cultivation to prepare LNTII.

In the first stage, the growth phase, the cells were cultured with glucose as the carbon source for the accumulation of biomass and enzymes until the cell growth reached the late logarithmic or stationary phase. The strains were streaked on solid medium and incubated at 30°C for 2-3 days. Single colonies were inoculated into 1.5 mL of liquid medium and shaken at 30°C, 200 rpm until OD₆₀₀ reached 1. This culture was then inoculated at a 2% volume into 5 L liquid medium in a 10 L fermenter and shaken overnight at 30°C, 200 rpm. Subsequently, it was inoculated at 2% volume into 50 mL liquid medium flasks and shaken at 30°C, 200 rpm for biomass accumulation.

In the second phase, the product synthesis phase, was the same as the second phase of Example 3.

The concentrations of LNTII in the fermentation broths of StrainKL-2-01 and StrainKM-2-01 were 4.21 mg/L and 4.25 mg/L, with lactose to LNTII conversion rates of 26.41% and 26.42%, respectively. The ethanol contents in the fermentation broth of the original starting strains were measured to be approximately 22.0 g/L and 22.2 g/L using a biosensor. The concentrations of LNTII in the fermentation broths of StrainKL-2-02 and StrainKM-2-02 were 7.18 g/L and 7.28 g/L, with lactose to LNTII conversion rates of 45.68% and 45.88%, respectively. The ethanol contents in the fermentation broths of the original starting strains were measured to be approximately 24.3 g/L and 24.1 g/L using a biosensor.

The media used in this example were as follows:
Solid medium: same as in Example 3.
Liquid medium: same as in Example 3.
Supplement: 2 g/L lactose, 20 g/L glucose.
Supplement: 2 g/L lactose, 20 g/L glucose, with final concentrations of 5 mM each of dipotassium hydrogen phosphate, magnesium sulfate, ammonium sulfate, and manganese sulfate.

### Example 17. Identification of the Synthetic Product

### (1) HPLC analysis of the compounds synthesized in Examples 4-9

The fermentation products of recombinant *Kluyveromyces lactis* and recombinant *Kluyveromyces marxianus* from Examples 4-9 were analyzed using HPLC under the previously mentioned conditions.

The retention time of the LNTII standard was 12.10 minutes.

The HPLC profiles in FIG. 2 demonstrate that the fermentation products of recombinant strains StrainKL-2-01, StrainKM-2-01, StrainKL-2-02 and StrainKM-2-02 in Examples 4 and 6, exhibited a strong peak at 12.10 minutes, which was consistent with the peak time of the LNTII standard.

The HPLC profiles in FIGs 3-4 clearly showed that the purified fermentation broths of the recombinant strains StrainKL-2-01, StrainKM-2-01, StrainKL-2-02 and StrainKM-2-02 in Examples 4 and 6 exhibited a strong absorption peak at 12.10 minutes, consistent with the retention time of the LNTII standard. This preliminary assessment suggests that the absorption peak at 12.10 minutes in Examples 4 and 6 may be the absorption peak of LNTII, indicating that LNTII was produced during the fermentation process.

The HPLC profiles of the purified fermentation broths from recombinant strains StrainKL-2-03 and StrainKM-2-03 in Example 5, and StrainKL-2-04 and StrainKM-2-04 in Example 7, were consistent with the HPLC profiles of the fermentation broths from the recombinant strains in Examples 4 and 6.

The comparative analysis of the HPLC profiles of the recombinant strains and the LNTII standard preliminarily indicated that LNTII was synthesized by the recombinant *Kluyveromyces lactis* and *Kluyveromyces marxianus* strains in Examples 4-7.

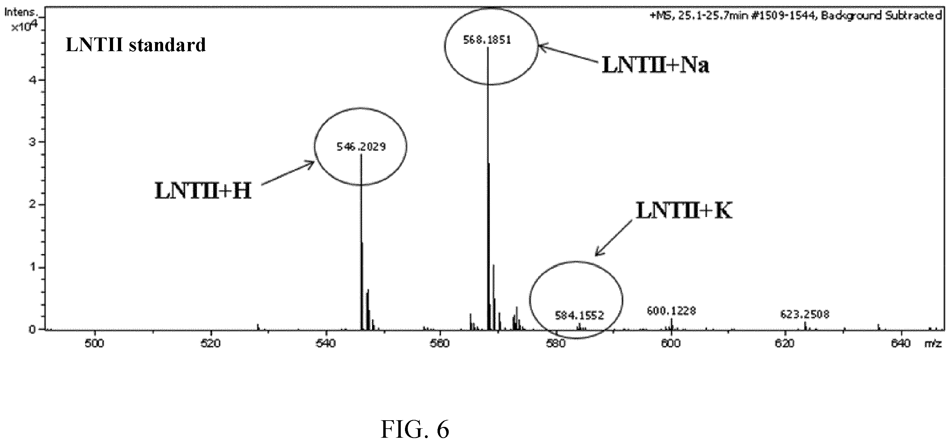

(2) FIG.6 showed the mass spectrometry for the components of the LNT-II standard and the synthetic products in Example 6 with the HPLC absorption peak at 12.10 min.

LNTII has a molecular formula of C₂₀H₃₅NO₁₆ with a molecular weight of 545.495. The molecular weights for LNTII+H, LNTII+Na, and LNTII+K are 546.2029, 568.485, and 584.593, respectively.

FIG. 5 indicated that the molecular weights of LNTII+H was 546.2029, that of LNTII+Na was 568.1851, and that of LNTII+K was 584.1552 based on the mass spectrum of LNTII standard.

FIG. 6 showed that the peaks with molecular weights of 546.2039, 568.1859, and 584.1559 were respectively close to the peaks with the molecular weights of LNTII+H, LNTII+Na, and LNTII+K in the standard LNTII based on the mass spectrum of the substance that had the absorption peak at 12.10 min in the fermentation products of StrainKL-2-02 and StrainKM-2-02 of Example 6.

In conclusion, the mass spectrometric analysis of the substance that had the absorption peak at 12.10 min in the fermentation products of Example 6 was corresponding to that of the LNTII standard, and the molecular weights were essentially consistent with theoretical values, indicating that the substance that had the absorption peak at 12.10 min in the fermentation products of Example 6 was LNTII.

Consequently, it was determined that the recombinant strains StrainKL-2-02 and StrainKM-2-02 indeed synthesized LNTII through combined HPLC and mass spectrometric analysis.

It can be inferred that the recombinant strains StrainKL-2-01, StrainKL-2-03, StrainKL-2-04, StrainKL-2-05, StrainKL-2-06, StrainKM-2-01, StrainKM-2-03, StrainKM-2-04, StrainKM-2-05, and StrainKM-2-06, which also showed absorption peaks at 12.10 minutes, synthesized the same substance as StrainKL-2-02 and StrainKM-2-02, indicating that StrainKL-2-01, StrainKL-2-02, StrainKL-2-03, StrainKL-2-04, StrainKL-2-05, StrainKL-2-06, StrainKM-2-01, StrainKM-2-02, StrainKM-2-03, StrainKM-2-04, StrainKM-2-05, and StrainKM-2-06 catalyzed the synthesis of LNTII.

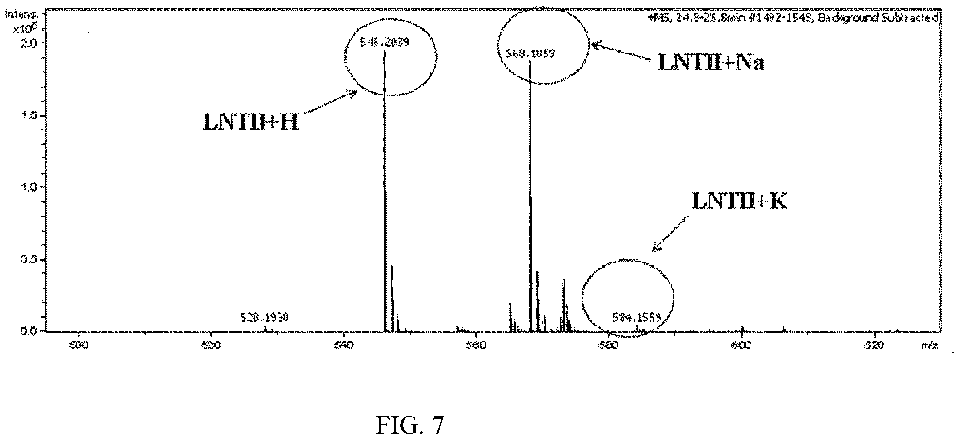

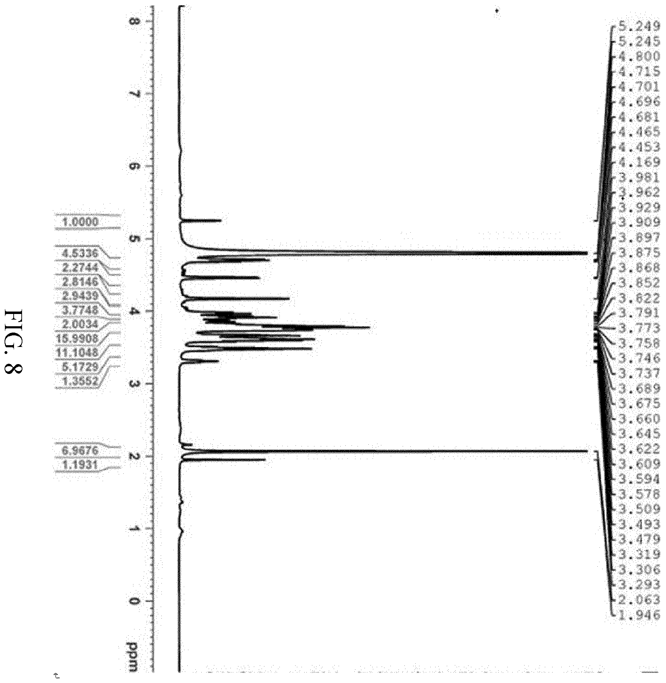

(3) NMR analysis was performed on the substance that had the absorption peak at 12.10 min in the fermentation products of Example 6, with results shown in FIGs 7 and 8.

The results were shown in FIGs 7 and 8. The coexistence of signals at 102.8 ppm in the ¹³C-NMR spectrum and 3.56 ppm in the ¹H-NMR spectrum indicated a β1-3 bond between GlcNAc and Gal residues. Similarly, signals at 102.8 ppm in the ¹³C-NMR spectrum and 3.48 ppm in the ¹H-NMR spectrum indicated a β1-4 bond between Gal and Glc residues. This further confirmed that the synthesized product from recombinant strains StrainKL-2-02 and StrainKM-2-02 was GlcNAc-β1-3Gal-β1-4Glc, also known as LNTII.

### Example 18. Comparison of growth curves between control strain StrainKL-1 and original strain StrainKL-0

Based on the experimental method for the first stage of the fermentation process (i.e. the cell growth stage) described in Example 3, 20 g/L lactose or a mixture of 20 g/L glucose with 20 g/L lactose was used as carbon sources. Samples were taken at 0, 12, 24, 36, 48, 60, and 72 hours to monitor the growth of control strain StrainKL-1 and original strain StrainKL-0. The results were recorded in Tables 6 and 7.

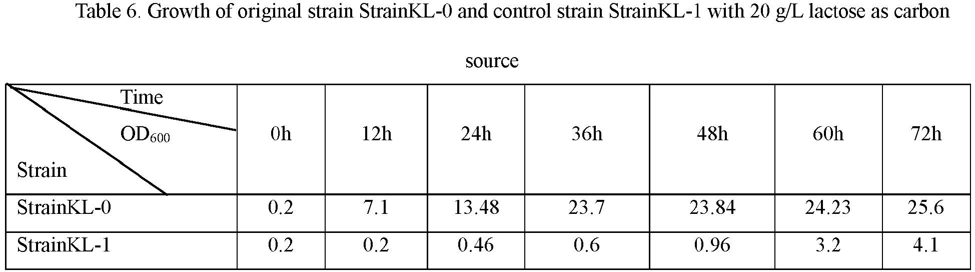

The data in Table 6 indicated that the growth of the control strain Strain KL-1 was significantly slower compared to the original strain Strain KL-0 when 20 g/L lactose was used as the carbon source.

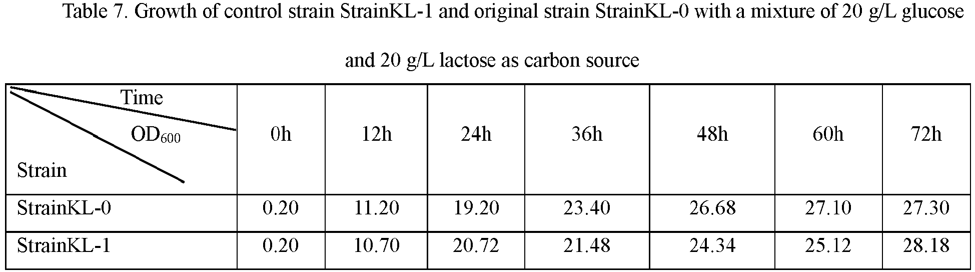

The data in Table 7 indicated that the growth rates of the control strain StrainKL-1 and the original strain StrainKL-0 were essentially identical when a mixture of 20 g/L glucose and 20 g/L lactose was used as the carbon source.

In summary, the data from Tables 6 and 7 indicated that the StrainKL-1 strain had a very low rate of lactose utilization, achieving the objective of the present invention to reduce lactose consumption.

In conclusion, the present invention for the first time discovered that LNTII can be synthesized by using the recombinant yeast with β-N-acetylglucosaminyl transferase. The recombinant yeast containing β-N-acetylglucosaminyl transferase gene demonstrated an exceptional capability in synthesizing LNTII. Among these, the recombinant yeast containing β-N-acetylglucosaminyl transferase gene from *Helicobacter pylori* showed a significantly higher rate of lactose-to-LNTII conversion compared to the recombinant yeast containing β-N-acetylglucosaminyl transferase gene from *Neisseria meningitidis* and *Salmonella enterica,* and the conversion rate of lactose-to-LNTII was also higher than that in *Escherichia coli.*

It should be noted that the examples provided above are intended to illustrate the technical solutions of the present invention and are not limiting. Despite detailed explanations of the present invention with given examples, those skilled in the art could modify or equivalently substitute the technical solutions of the present invention as needed, without deviating from the spirit and scope of the present invention.

### (The original is in electronic form)

| | | |
|---|---|---|
| 0-1 | PCT/RO134 Form | |
| 0-1-1 | Indications relating to deposited microorganism | |
| | Or other biological material (PCT Rule 13*bis*) | |
| | Software version | i-system |
| | | Version 1.0.9 20230217 MT/FOP 20140331/0.20.5.21 |
| 0-2 | International application No. | PCT/CN2023/077280 |
| 0-3 | Applicant's or agent's file reference | PCT202302 |
| | | |
| 1 | The indications made below relate to the deposited microorganism or other biological material referred to in the description | |
| 1-1 | Page | 4 |
| 1-2 | Line | |
| 1-3 | Identification of deposit | |
| 1-3-1 | Name of depositary institution | CCTCC CHINA CENTER FOR TYPE CULTURE COLLECTION |
| 1-3-2 | Address of depositary institution | 430072, College of Life Sciences, Wuhan University, Wuhan City, Hubei Province, China |
| 1-3-3 | Date of deposit | 23 February 2022 (23.02.2022) |
| 1-3-4 | Accession Number | CCTCC NO: M 2022118 |
| 1-4 | Additional indications | Viable |
| 1-5 | Designated states for which indications are made | For all designated States |
| 1-6 | Separate furnishing of indications | |
| | The indications listed below will be submitted to the International Bureau later | |
| For receiving Office use only | | |
| 0-4 | This sheet was received with the international application (Yes or No) | |
| 0-4-1 | Authorized officer | |
| For International Bureau use only | | |
| 0-5 | This sheet was received by the International Bureau on | |
| 0-5-1 | Authorized officer | |

## Claims

1. An enzyme for catalyzing the production of lacto-N-triose II (LNTII), classified according to the Carbohydrate-Active Enzymes (CAZy) database as belonging to the glycoside hydrolase family 28, wherein the enzyme is a β-N-acetylglucosaminyl transferase.

2. The enzyme according to claim 1, comprising an amino acid sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity to SEQ ID NO: 1, SEQ ID NO: 2, or SEQ ID NO: 3, and possessing the activity to catalyze the production of LNTII.

3. The enzyme according to claims 1 or 2, wherein the enzyme is a β-N-acetylglucosaminyl transferase originating from *Neisseria meningitidis;*
preferably, an amino acid sequence of the β-N-acetylglucosaminyl transferase is as set forth in SEQ ID NO:1, or comprises an amino acid sequence as set forth in SEQ ID NO:1, or has at least 70% amino acid sequence identity with SEQ ID NO: 1, or comprises an amino acid sequence with at least 70% sequence identity with SEQ ID NO: 1.

4. The enzyme according to claims 1 or 2, wherein the enzyme is a β-N-acetylglucosaminyl transferase originating from *Helicobacter pylori;*
preferably, an amino acid sequence of the β-N-acetylglucosaminyl transferase is as set forth in SEQ ID NO:2, or comprises an amino acid sequence as set forth in SEQ ID NO:2, or has at least 70% amino acid sequence identity with SEQ ID NO:2, or comprises an amino acid sequence as set forth in SEQ ID NO:2, or comprises an amino acid sequence with at least 70% sequence identity with SEQ ID NO:2.

5. The enzyme according to claims 1 or 2, wherein the enzyme is a β-N-acetylglucosaminyl transferase originating from *Salmonella enterical*; an amino acid sequence of the β-N-acetylglucosaminyl transferase is as set forth in SEQ ID NO:3, or has at least 70% amino acid sequence identity with SEQ ID NO:3, or comprises an amino acid sequence as set forth in SEQ ID NO:3, or comprises an amino acid sequence with at least 70% sequence identity with SEQ ID NO:3.

6. A recombinant yeast for producing lacto-N-triose II (LNTII), comprising an amino acid sequence of β-N-acetylglucosaminyl transferase as claimed in any one of claims 1-5 and a nucleic acid sequence encoding the amino acid sequence;
preferably, the recombinant yeast is a yeast cell, selected from genera *Candida, Hansenula, Kluyveromyces, Pichia, Saccharomyces, Schizosaccharomyces,* or *Yarrowia;* more preferably, the yeast cell is *Saccharomyces cerevisiae* cell, *Kluyveromyces lactis* cell, *Kluyveromyces marxianus* cell, *Yarrowia lipolytica* cell, *Rhodotorula graminis* cell, *Saccha romyces pastorianus* cell;
preferably, the recombinant yeast is recombinant *Kluyveromyces;* preferably, the recombinant yeast is recombinant *Kluyveromyces lactis* or *Kluyveromyces marxianus.*

7. The recombinant yeast according to claim 6, further comprising a marker gene with disruption;
preferably, the disruption refers to a partial disruption of the marker gene;
preferably, the marker gene comprises a nucleotide sequence encoding an amino acid sequence of entire or partial of β-galactosidase, the amino acid sequence having at least 50%, 51%, 52%, 53%, 54%, 55%, 60%, 61%, 62%, 63%, 64%, 65%, 66%, 67%, 68%, 69%, 70%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity to SEQ ID NO:4 and/or SEQ ID NO:5;
the recombinant yeast is a recombinant *Kluyveromyces lactis* cell; preferably, the recombinant yeast comprises at least one marker gene that is disrupted, and comprises an amino acid sequence of the marker, and a nucleic acid sequence encoding the marker;
the recombinant yeast is a cell of recombinant *Kluyveromyces marxianus;* preferably, the recombinant yeast comprises at least one marker gene that is disrupted, and comprises an amino acid sequence of the marker, and a nucleic acid sequence encoding the marker.

8. The recombinant yeast according to claims 6 or 7, wherein the LNTII is produced while concurrently fermenting the recombinant yeast to produce ethanol;
preferably, a method for constructing the recombinant yeast comprises: (a) cultivating a parental strain; (b) disrupting a marker gene in the parental strain mentioned in (a); (c) introducing an enzyme capable of producing LNTII; and preferably further comprising the step of (d) isolating the recombinant yeast.

9. A use of a recombinant yeast according to claims 6 or 7 for producing LNTII;
the use of the recombinant yeast for producing LNTII, preferably, comprises: (a) culturing the recombinant yeast cells in a fermentable culture medium under suitable conditions to produce LNTII; and (b) recovering the LNTII;
the use of the recombinant yeast for producing LNTII, preferably, the fermentable culture medium is an aqueous solution;
the use of the recombinant yeast for producing LNTII, preferably, the fermentable culture medium is an aqueous solution comprising lactose and/or glucose and/or fructose and/or sucrose and/or galactose;
the use of the recombinant yeast for producing LNTII, preferably, a temperature for culturing the recombinant yeast is 15-60°C;
the use of the recombinant yeast for producing LNTII, preferably, wherein the temperature for culturing the recombinant yeast is 28-32°C;
the use of the recombinant yeast for producing LNTII, preferably, the fermentable culture medium also comprises inorganic salts and/or trace elements;
the use of the recombinant yeast for producing LNTII, preferably, the inorganic salts and/or trace elements are selected from dipotassium phosphate, magnesium sulfate, ammonium sulfate, manganese sulfate, potassium sulfate, or any combination thereof;
the use of the recombinant yeast for producing LNTII, preferably, wherein a final concentration of the inorganic salts or trace elements is 3-7mM;
the use of the recombinant yeast for producing LNTII, preferably, the LNTII is obtained after separation and purification by a method of adding activated carbon.

10. An ultraviolet-induced mutant of a recombinant yeast as claimed in claims 6 or 7, having further enhanced capability for synthesizing LNTII.
